# EUROPEAN PATENT APPLICATION

(11) **EP 4 289 929 A1**
(43) Date of publication of application: **13.12.2023**
(21) Application number: 22177377.3
(22) Date of filing: 06.06.2022
(51) Int. Cl.: C12M 3/00, C12M 3/06, C12M 1/42

(54) **MEMBRANE STRUCTURE FOR STIMULATING AND SENSING BIOLOGICAL MATERIALS, AND CELL CULTURE PLATE AND MICROFLUIDIC DEVICE BOTH USING THE SAME**

(71) Applicant: Finnadvance Oy, 90220 Oulu (FI)
(72) Inventor: Singh, Prateek, 90220 Oulu (FI); Nguyen, Hoang Tuan, 90220 Oulu (FI)
(74) Representative: Papula Oy

(57) **Abstract**

The present disclosure relates generally to the field of biotechnology and, in particular, to a membrane structure for stimulating and sensing a biological material. The membrane structure comprises a porous membrane having at least one array of needles provided on its top surface and/or bottom surface, without closing through pores of the membrane. Each needle of the at least one array of needles is configured to penetrate the biological material, is made of a non-conducting material and at least partly coated with a conducting electrode. By using the membrane structure thus configured, it is possible to apply an electrical stimulus to the biological material and take response measurements. Moreover, the presence of the through pores in the membrane structure allows the biological material (penetrated by the needles) to be perfused or vascularized through a microfluidic channel with which the membrane structure may be brought into contact. In other embodiments, a cell culture plate and a microfluidic device are provided, each of which incorporates the membrane structure.

## Description

### TECHNICAL FIELD

The present disclosure relates generally to the field of biotechnology. In particular, the present disclosure relates to a membrane structure adapted for stimulating and sensing biological materials (e.g., organoids, spheroids, tissue slices, cell layers, etc.), as well as to a cell culture plate and a microfluidic device both comprising the membrane structure.

### BACKGROUND

In recent years, 'organoid-on-a-chip' systems have been developed, which support organoid culture, perfusion and vascularization by means of microfluidic channels carrying a cell culture medium for organoids. The 'organoid-on-a-chip' systems are very different in design and operational principle and are intended to significantly affect the future of medicine (e.g., they can be used in drug development and on-chip disease model investigation).

Reproducing and optimizing a microenvironment is the tenet to establishing *in-vitro* organoids. In the existing 'organoid-on-a-chip' systems, the formation of organoids usually relies on a random self-assembly or self-organization in the cell culture medium, and there is no possibility of using different stimuli (e.g., an electric pulse that mimics a nerve pulse for a brain organoid) which are common *in vivo.* Therefore, more engineering is required to promote the culture of organoids and any similar or other biological materials *in vitro.*

### SUMMARY

This summary is provided to introduce a selection of concepts in a simplified form that are further described below in the detailed description. This summary is not intended to identify key features of the present disclosure, nor is it intended to be used to limit the scope of the present disclosure.

It is an objective of the present disclosure to provide a technical solution that allows electrical stimulation of biological materials (e.g., organoids, spheroids, tissue slices, cell layers, etc.) and associated response measurements.

The objective above is achieved by the features of the independent claims in the appended claims. Further embodiments and examples are apparent from the dependent claims, the detailed description and the accompanying drawings.

According to a first aspect, a membrane structure for stimulating and sensing a biological material is provided. The membrane structure comprises a membrane having through pores formed therein. The membrane has a top surface and a bottom surface. The membrane structure further comprises at least one array of needles provided on at least one of the top surface and the bottom surface of the membrane such that the through pores of the membrane remain open. Each needle of the at least one array of needles is shaped to penetrate the biological material when the biological material is provided on the at least one array of needles. Furthermore, each needle of the at least one array of needles is made of a non-conducting material and at least partly coated with a conducting electrode. By using the membrane structure thus configured, it is possible to apply an electrical stimulus to the biological material and take response measurements. Moreover, if the biological material is represented, for example, by one or more organoids, the presence of the through pores in the membrane structure allows the organoid(s) penetrated by the needles to be perfused or vascularized through a microfluidic channel with which the membrane structure may be brought into contact.

In one exemplary embodiment of the first aspect, at least one needle of the at least one array of needles is fully coated with the conducting electrode. If each of the needles is fully coated with the conducting electrode, it is possible to provide strong electrical stimulation throughout the biological material. If only some of the needles are fully coated with the conducting electrodes, it is possible to provide strong localized electrical stimulation in individual points of the biological material, which may be beneficial in some scenarios (e.g., when the strong electrical stimulation throughout the biological material is undesirable, since it may lead to damage to the biological material).

In one exemplary embodiment of the first aspect, each needle of the at least one array of needles comprises a plurality of portions spaced from each other along or across the needle. Each portion of the plurality of portions is coated with the conducting electrode. With this configuration, each needle penetrating the biological material may provide electrical stimulation of the biological material simultaneously at different locations along the needle length rather than continuously along the needle length. For example, this needle configuration may prevent excessive heating of the biological material. Moreover, since only some portions of each needle are coated with the conducting electrode, it is possible to reduce electrode-material consumption.

In one exemplary embodiment of the first aspect, the plurality of portions of each needle of the at least one array of needles has an equal inter-portion spacing. This needle configuration may provide uniform electrical stimulation along the needle length.

In one exemplary embodiment of the first aspect, the plurality of portions of each needle of the at least one array of needles has a varying inter-portion spacing. This needle configuration may be used when it is required to provide non-uniform electrical stimulation along the needle length (e.g., the inter-portion spacing may gradually decrease towards the tip of each needle).

In one exemplary embodiment of the first aspect, the conducting electrode is configured as a ring surrounding each portion of the plurality of portions. Such a ring-like electrode is easy to implement. Moreover, it may provide omnidirectional propagation of an electrical stimulus from each portion of the plurality of portions of each needle, thereby uniformly affecting the biological material in the vicinity of each portion of the plurality of portions of each needle.

In one exemplary embodiment of the first aspect, the at least one array of needles comprises a single array of needles each having a tapered shape (e.g., a conical shape), a mushroom-like shape, or a uniform cross-section shape (e.g., a cylindrical shape). The needles having such shapes may easily penetrate different biological materials (e.g., organoids, spheroids, tissue slices, cell layers, etc.).

In one exemplary embodiment of the first aspect, the at least one array of needles comprises a first array of needles and a second array of needles. The first array of needles and the second array of needles differ from each other in at least one of a needle size, a needle shape and a needle spacing. By having two different arrays of needles, it is possible to affect (by means of electrical stimulation) one or more biological materials of interest differently, which may be useful in some applications. In other words, it is possible to create different "gradients" of electrical stimulation across the biological material(s). Moreover, if it is required to perform, for example, the electrical stimulation of two differently sized organoids, then the array of shorter (and/or closely spaced) needles may be used for the smaller organoid, while the array of longer (and/or widely spaced) needles may be used for the bigger organoid.

In one exemplary embodiment of the first aspect, each of the first array of needles and the second array of needles has one of the following needle shapes: a tapered shape, a mushroom-like shape, and a uniform cross-section shape. The needles having such shapes may easily penetrate different biological materials (e.g., organoids, spheroids, tissue slices, etc.).

In one exemplary embodiment of the first aspect, the first array of needles and the second array of needles are provided on one of the top surface and the bottom surface of the membrane. In this configuration, it is possible to perform the electrical stimulation of (e.g., differently sized) biological materials (simultaneously or in sequence) on one surface of the membrane structure.

In one exemplary embodiment of the first aspect, the first array of needles and the second array of needles are non-overlapping. For example, the first array of needles may be fully arranged on the left side of the top surface of the membrane, while the second array of needles may be fully arranged on the right side of the top surface of the membrane, or vice versa. With this non-overlapping arrangement, it is possible to affect (by means of electrical stimulation) the same biological materials simultaneously by using the different first and second arrays of needles. For example, the same organoids may be provided on the first and second arrays of needles, whereupon they may be stimulated and sensed in parallel. By so doing, it is possible to reduce the overall time of research.

In one exemplary embodiment of the first aspect, the first array of needles and the second array of needles are arranged alternately. This may allow one to affect (by means of electrical stimulation) the biological material by using the first and second arrays of needles alternately.

In one exemplary embodiment of the first aspect, each of the first array of needles and the second array of needles is a 2D array of needles. In this embodiment, the first array of needles and the second array of needles are provided in a staggered arrangement. By using the staggered arrangement, it is possible to increase the efficiency of affecting (by means of electrical stimulation) the biological material by using the first and second arrays of needles alternately.

In one exemplary embodiment of the first aspect, the first array of needles is provided on the top surface of the membrane, while the second array of needles is provided on the bottom surface of the membrane. In this configuration, it is possible to perform the electrical stimulation of (e.g., differently sized) biological materials on both surfaces of the membrane structure. This may allow one to increase the number of biological materials (e.g., organoids) which may be exposed to the electrical stimulation at the same time.

According a second aspect, a cell culture plate is provided. The plate comprises a housing encompassing a plurality of wells. Each well of the plurality of wells is provided with a cell culture medium and a replaceable insert configured to be immersed into the cell culture medium. Each of the replaceable inserts comprises the membrane structure according to the first aspect. With such a cell culture plate, it is possible to perform the electrical stimulation of biological materials (e.g., organoids, spheroids, tissue slices, cell layers, etc.) in the wells by applying electrical signals to the conducting electrodes of the membrane structures, while studying the interaction between the biological materials and cells from the cell culture medium.

According to a third aspect, a microfluidic device is provided. The device comprises a housing having a microfluidic channel and a top-loaded chamber. The microfluidic channel is configured to pass a fluid or cell culture medium therethrough. The device further comprises the membrane structure according to the first aspect, which is arranged in the housing such that the top-loaded chamber is in fluid communication with the microfluidic channel via the through pores of the membrane structure. By using the microfluidic device thus configured, it is possible to perform the electrical stimulation of one or more biological materials (e.g., organoids placed on the needles through the top-loaded chamber) by applying electrical signals to the conducting electrodes of the membrane structure, while providing the perfusion or vascularization of the biological materials through the microfluidic channel.

In one exemplary embodiment of the third aspect, the top-loaded chamber has an additional microfluidic channel formed therein. The additional microfluidic channel is configured to pass an additional fluid or cell culture medium therethrough. In this embodiment, the biological material(s) provided on the at least one array of needles may also be perfused or vascularized from another (top) side.

Other features and advantages of the present disclosure will be apparent upon reading the following detailed description and reviewing the accompanying drawings.

### BRIEF DESCRIPTION OF THE DRAWINGS

The present disclosure is explained below with reference to the accompanying drawings in which:
FIG. 1 shows a schematic cross-sectional view of a membrane structure for stimulating and sensing a biological material in accordance with one exemplary embodiment;
FIG. 2 shows a schematic cross-sectional view of an organoid penetrated by an array of needles included in the membrane structure shown in FIG. 1;
FIG. 3 shows a schematic perspective view of a cell culture plate in accordance with one exemplary embodiment;
FIG. 4 shows a schematic cross-sectional view of one well of the cell culture plate, as taken along line A-A of FIG. 3; and
FIG. 5 shows a block-scheme of a microfluidic device or chip in accordance with one exemplary embodiment.

### DETAILED DESCRIPTION

Various embodiments of the present disclosure are further described in more detail with reference to the accompanying drawings. However, the present disclosure may be embodied in many other forms and should not be construed as limited to any certain structure or function discussed in the following description. In contrast, these embodiments are provided to make the description of the present disclosure detailed and complete.

According to the detailed description, it will be apparent to the ones skilled in the art that the scope of the present disclosure encompasses any embodiment thereof, which is disclosed herein, irrespective of whether this embodiment is implemented independently or in concert with any other embodiment of the present disclosure. For example, apparatuses disclosed herein may be implemented in practice using any numbers of the embodiments provided herein. Furthermore, it should be understood that any embodiment of the present disclosure may be implemented using one or more of the elements presented in the appended claims.

The word "exemplary" is used herein in the meaning of "used as an illustration". Unless otherwise stated, any embodiment described herein as "exemplary" should not be construed as preferable or having an advantage over other embodiments.

Any positioning terminology, such as "left", "right", "top", "bottom", "above", "under", etc., may be used herein for convenience to describe one element's or feature's relationship to one or more other elements or features in accordance with the figures. It should be apparent that the positioning terminology is intended to encompass different orientations of the structure and device disclosed herein, in addition to the orientation(s) depicted in the figures. As an example, if one imaginatively rotates the structure or device in the figures 90 degrees clockwise, elements or features described as "top" and "bottom" relative to other elements or features would then be oriented, respectively, "right" and "left" relative to the other elements or features. Therefore, the positioning terminology used herein should not be construed as any limitation of the present disclosure.

Furthermore, although the numerative terminology, such as "first", "second", etc., may be used herein to describe various elements or features, these elements or features should not be limited by this numerative terminology. This numerative terminology is used herein only to distinguish one element or feature from another element or feature. For example, a first array of needles discussed below could be called a second array of needles, and vice versa, without departing from the teachings of the present disclosure.

In the embodiments disclosed herein, a cell may refer to a biological cell, such as a plant cell, an animal cell (e.g., a mammalian cell), a bacterial cell, a fungal cell, or the like. A mammalian cell may be, for example, from a human, a mouse, a horse, a goat, a sheep, a cow, a primate, or the like.

As used in the embodiments disclosed herein, a biological material may refer to a microorganism, a cell layer, a cell system, a molecule (e.g., DNA, RNA, cDNA), a protein, a peptide, an enzyme, an organoid, a spheroid, an organ, a tissue slice or fragment (either in a suspension or a hydrogel), any other organic matter or biologically active compound, or any combination of the aforesaid. Although a reference is made below to organoids, this should not be construed as any limitation of the present disclosure; any other of the examples given above may be equally used as the biological material in the embodiments disclosed herein.

As used in the embodiments disclosed herein, an organoid may refer to a 3D multicellular in *vitro* tissue construct that mimics its corresponding *in vivo* organ (e.g., the brain, the stomach, or the liver), such that it may be used to study different aspects of that organ. Such tissue constructs are usually derived from stem cells which may be either pluripotent (embryonic or induced) or adult stem cells from various organs. The processes that form these tissue constructs *in vitro* are well-known in this technical field, for which reason their description is omitted herein. Such organoid formation processes are believed to approximate natural development or tissue maintenance. The developmental potential of the starting stem cells will influence how complex the organoid may be.

In the embodiments disclosed herein, a cell culture medium, also referred to as a growth medium, may refer to a liquid, suspension, or gel designed to support cellular growth *in vitro.* There are different types of culture media suitable for growing different types of cells. In general, the culture media may be broken into two primary categories: natural media and synthetic media. The natural media are those that are derived from tissue extraction or animal body fluids, such as plasma, lymph, and serum. The synthetic media are those created using a variety of organic and inorganic compounds. Moreover, the cell culture medium itself may comprise cells, bodies of cells (e.g., organoids), lipid particles, including natural or engineered (e.g., exosomal microvesicles, vacuoles, micelles or lipid particles of various design, virus particles, nanoparticles, etc.).

As used in the embodiments disclosed herein, a microfluidic device (also referred to as a microfluidic chip in this technical field) may refer to a device having various wells (e.g., reservoirs, chambers, etc.) connected by one or more microfluidic channels in which fluids (i.e., culture media) will exhibit microfluidic behavior in their flow through the microfluidic channels. Each microfluidic channel of the microfluidic device may relate to a sub-millimeter-scale channel that has a hydraulic diameter below 1 mm and is used for fluidically connecting the wells of the microfluidic channel. In other words, the microfluidic channel generally denotes a channel configured to pass different fluids, such, for example, as culture media (e.g., cell suspensions), reagents, or gels, in micro-scale volumes. The microchannel may be shaped such that it has appropriate flow characteristics (e.g., flow rates) depending on particular applications. For example, the microchannel may have a rectangular, e.g., square, or rounded cross-section, as well as be straight, bended, or tilted towards a required direction. In general, the microfluidic cell culture performed by the microfluidic device is generally related to cell culture, maintenance and perturbation in micro-scale fluid volumes.

The exemplary embodiments disclosed herein provide a technical solution that allows mitigating or even eliminating the drawbacks of the prior art. In particular, the technical solution disclosed herein provides a membrane structure for stimulating and sensing a biological material. The membrane structure comprises a porous membrane having at least one array of needles provided on its top surface and/or bottom surface, without closing the through pores of the membrane. Each needle of the at least one array of needles is configured to penetrate the biological material, is made of a non-conducting material and at least partly coated with a conducting electrode. By using the membrane structure thus configured, it is possible to apply an electrical stimulus to the biological material and take response measurements. Moreover, the presence of the through pores in the membrane structure allows the biological material (penetrated by the needles) to be perfused or vascularized through a microfluidic channel with which the membrane structure may be brought into contact.

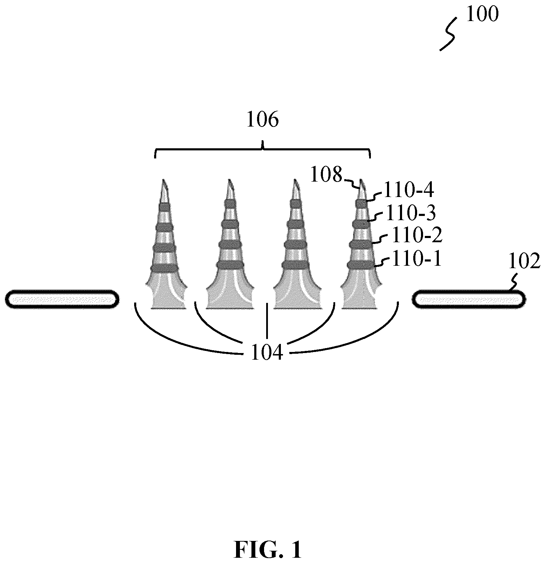

FIG. 1 shows a schematic cross-sectional view of a membrane structure 100 for stimulating and sensing a biological material in accordance with one exemplary embodiment. As shown in FIG. 1, the membrane structure 100 comprises a membrane 102 with through pores 104 formed therein (e.g., by using at least one of particle/fluid bombardment, etching and laser burning). The membrane 102 may be made (e.g., by using 3D printing, casting, micromolding, injection molding, etc.) of one of the following materials: polypropylene, polycarbonate, polyethersulfone, polyethylene terephthalate, polyimide, polytetrafluoroethylene (PTFE), silicone (e.g., commercially available sylgard, room-temperature-vulcanizing (RTV) silicone, elastosil), matrigel, collagen, extracellular matrices, and silicon nitride. As for the through pores 104, they may have a different cross-sectional shape, such, for example, as triangular, rectangular, square, oval, or polygonal (e.g., hexagonal). The polygonal cross-sectional shape of the through pores 104 may refer to a convex or concave polygon. In some embodiments, the through pores 104 may have different cross-sectional shapes, such as any combination of two or more of the above-mentioned cross-sectional shapes (e.g., the combination of rectangular and oval cross-sectional shapes). It should be also noted that each of the through pores 104 may have a pore size varying within a predefined range of values, and a spacing (pitch) between the through pores 104 may vary within the same predefined range of values. For example, the pore size and spacing may both vary from 200 nanometers to 30 micrometers. It should be apparent that a pore density will depend on the pore size and spacing selected depending on particular applications. The pore density may, for example, be from 10³ to 10⁷ pores per millimeter square. In general, the pore size, spacing and density may be selected depending on particular applications. For example, if the biological material is represented by one or more organoids placed on the top surface of the membrane 102 and the bottom surface of the membrane 102 is in contact with a microfluidic channel, the pore size, spacing and density may be selected such that the organoid(s) is(are) properly perfused or vascularized through the microfluidic channel. In another example, if the organoid(s) is(are) again placed on the top surface of the membrane 102 but the bottom surface of the membrane 102 is in contact with a cell culture medium contained in an individual well, the pore size, spacing and density may be selected such that the proper interaction between the organoid(s) and the cells from the cell culture medium is provided.

Referring back to FIG. 1, the membrane structure 100 further comprises an array 106 of needles provided on the top surface of the membrane 102 such that the through pores 104 remain open. Each needle 108 of the array 106 of needles is made (e.g., by using 3D printing in-situ, micromolding, injection molding in situ) of a non-conducting material, such as silicone, plastic or ceramic. The non-conducting material may also be the same as the membrane material. Each needle 108 of the array 106 of needles may be shaped such that it may penetrate or pierce the biological material placed thereon. The size and shape of each needle 108 and the spacing between the needles 108 may be selected based on the type and size of the biological material (e.g., an organoid) to be electrically stimulated. As shown in FIG. 1, each needle 108 of the array 106 of needles has a conical shape. In other embodiments, the needles 108 may have any other tapered shape, or a mushroom-like shape, or a uniform cross-section shape (e.g., a cylindrical shape). As for the sizes of the needles 108, they may have a length of 50 to 3000 micrometers, and a diameter of 7 to 800 micrometers.

To provide the possibility of applying an electrical stimulus to the biological material, each needle 108 of the array 106 of needles comprises four evenly spaced portions 110-1, 110-2, 110-3 and 110-4, each of which is provided with a conducting electrode. The conducting electrode may be made of a metal (e.g., gold, silver, aluminum, copper, titanium, silver, nickel, gallium, indium, etc.), a metal alloy, a conducting polymer, a conducting co-polymer (e.g., graphene, carbon nanotubes, Poly(3,4-ethylenedioxythiophene and their derivatives, etc.), or a semiconductor material belonging to groups III-V of Mendeleev's periodic table (e.g., Ge, Si, etc.). Given the shapes of the portions 110-1, 110-2, 110-3 and 110-4, each conducting electrode has a ring-like shape. The conducting electrodes may be implemented on the portions 110-1, 110-2, 110-3 and 110-4 by means of photolithography (e.g., electron beam lithography), sputtering or physical vapor deposition (e.g., electron beam evaporation). The ring-like shape may be a circular ring, a square ring, a triangular ring, an oval ring, and so on, depending on the cross-sectional shape of each needle 108. The conducting electrodes may be coupled (e.g., by means of wires) to a measurement circuit (not shown in FIG. 1) configured to feed electrical signals to the conducting electrodes, thereby providing the electrical stimulation of the biological material. The measurement circuit is further configured to sense the biological material, i.e., receive a response signal from the biological material via the conducting electrodes.

The number, arrangement and shape of the needles 108 and the portions 110-1, 110-2, 110-3 and 110-4 are shown in FIG. 1 for illustrative purposes only and should not be construed as any limitation of the present disclosure. In some embodiments, each needle 108 may comprise a smaller or bigger number of portions which are evenly or unevenly spaced from each other along the microneedle length and coated with the conducting electrodes; in these embodiments, the spatial distribution of such electrode-coated portions along the length of each needle 108 may be the same (i.e., the electrode-coated portions of the needles 108 are aligned with each other, as shown in FIG. 1), or the spatial distribution of the electrode-coated portions in one half of the needles 108 may differ from that in another half of the needles 108. The portions may also be evenly or unevenly spaced from each other across each needle 108 (i.e., across the width of the needle 108) and coated with the conducting electrodes (e.g., if each needle 108 has a cylindrical shape, there may be two opposite, electrode-coated portions on the lateral surface of the cylindrical needle). In other embodiments, one or more of the needles 108 may be fully coated with the conducting electrode, so that such needle(s) 108 has (have) no exposed (i.e., uncoated) portion.

Furthermore, there may be two or more arrays of needles formed on one (top or bottom) surface or both (top and bottom) surfaces of the membrane 102, which may differ from each other in at least one of the following: a needle size, a needle shape and an inter-needle spacing. Being formed on one surface of the membrane 102, such arrays of needles may be non-overlapping (e.g., in case of two arrays of needles, one of them may be fully arranged on the left side of the top surface of the membrane 102, while another may be fully arranged on the right side of the top surface of the membrane 102, or vice versa), or may be arranged alternately (e.g., in case of two 2D arrays of needles, they may be arranged in a staggered arrangement).

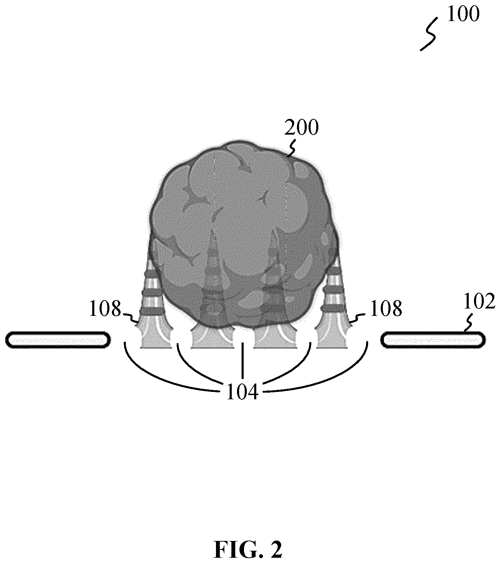

FIG. 2 shows a schematic cross-sectional view of an organoid 200 penetrated by the array 106 of needles of the membrane structure 100. As shown in FIG. 2, the organoid 200 has a substantially circular cross-section, for which reason the two central needles 108 are fully inside the organoid 200, while the two side needles 108 have only their tips penetrating the organoid 200. This circumstance may be considered when selecting the number and spatial distribution of the electrode-coated portions 110-1, 110-2, 110-3 and 110-4 for each needle 108. For example, each of the two side needles 108 may be designed such that it comprises only one electrode-coated portion in proximity of its tip, while each of the two central needles 108 may comprise a bigger number of closely spaced electrode-coated portions.

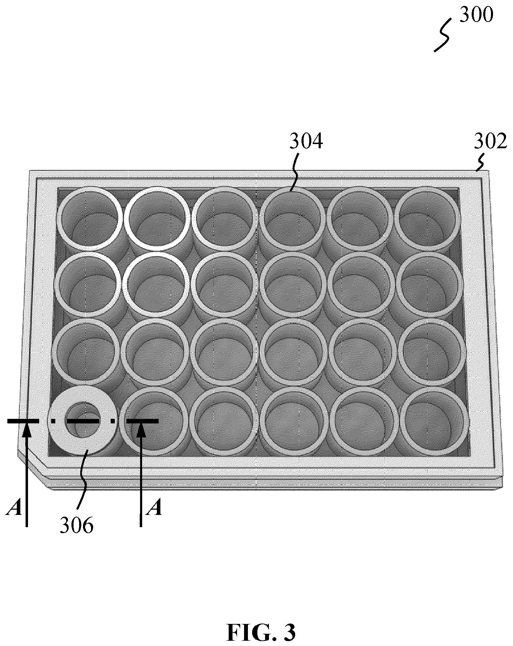

FIG. 3 shows a schematic perspective view of a cell culture plate 300 in accordance with one exemplary embodiment. As shown in FIG. 3, the cell culture plate 300 comprises a (e.g., rigid) housing 302 encompassing a plurality of wells 304. Each well 304 is shaped as a cylinder and is provided with a cell culture medium and a replaceable insert 306 configured to be immersed into the cell culture medium. Each of the inserts 306 may comprise the membrane structure 100. The cell culture plate 300 makes it possible to study the interaction between the biological material(s) (e.g., organoid(s)) and cells of desired type(s) simultaneously and under the same conditions. It should be apparent to those skilled in the art that the number, shape and spacing of the wells 304 shown in FIG. 3 are for illustrative purposes only and should not be construed as any limitation of the present disclosure. For example, the cell culture plate 302 may be configured to fit the standard 96, 384, or 1536 microtiter plates.

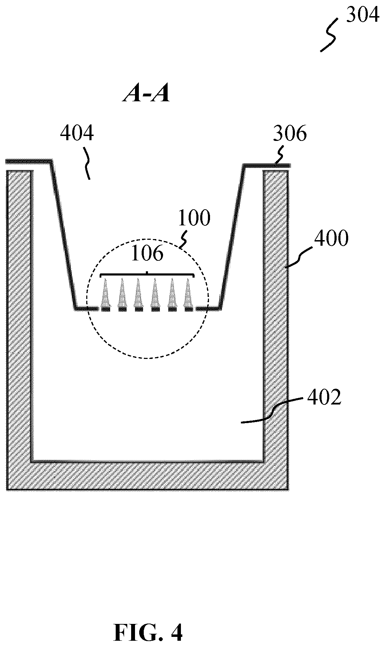

FIG. 4 shows a schematic cross-sectional view of one well 304 of the cell culture plate 300, as taken along line A-A of FIG. 3. As shown in FIG. 4, the well 304 comprises a body 400 with an inner cavity 402 which is intended to be filled with the cell culture medium. The insert 306 comprises the membrane structure 100 attached to its bottom and is immersed in the inner cavity 402 such that the membrane structure 100 is at least partly in contact with the cell culture medium (e.g., only the bottom surface of the membrane structure 100 is in contact with the cell culture medium). The insert 306 is provided with an inner cavity 404. The inner cavity 404 may be used, for example, to place different organoids on the top side of the membrane structure 100 (i.e., on the array 106 of needles) for research purposes. It should be again noted that the membrane structure 100 may comprise the same or different array of needles on its bottom side, so that it is possible to place the organoid(s) on such a bottom array of needles and fully immerse it in the cell culture medium.

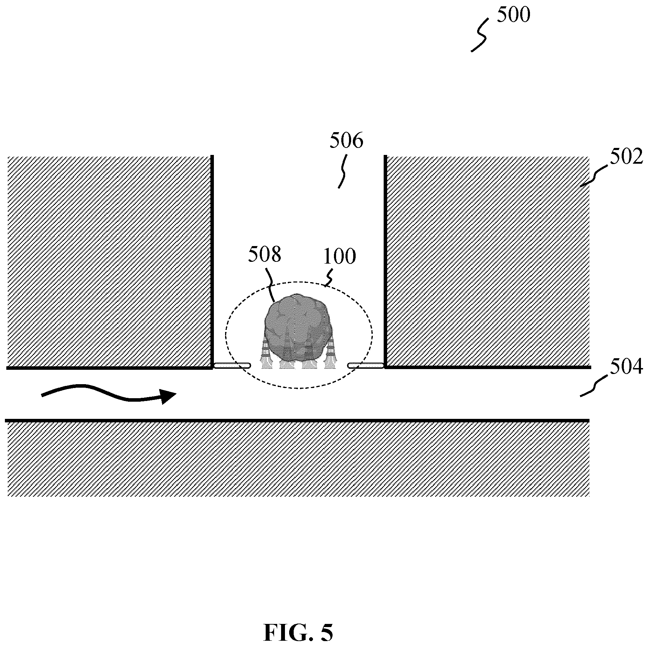

FIG. 5 shows a block-scheme of a microfluidic device or chip 500 in accordance with one exemplary embodiment. As shown in FIG. 5, the device 500 comprises a (e.g., rigid) housing 502, a microfluidic channel 504, the membrane structure 100 brought into contact with the microfluidic channel 504, and a top-loaded chamber 506 which is in fluid communication with the microfluidic channel 504 via the through pores of the membrane structure 100. An organoid 508 may be placed on the top surface of the membrane 102 such that it is penetrated by the array 106 of needles. The microfluidic channel 504 is configured to pass a fluid or cell culture medium therethrough, which is schematically shown by the curved arrow in FIG. 5. The fluid or cell culture medium may comprise one or more nutrient substances and/or other substances necessary for keeping the organoid 508 alive. Thus, the organoid 508 may be perfused and vascularized through the microfluidic channel 504 on the bottom side of the membrane 102. Although the device 500 is shown to have one microfluidic channel 504 and/or one top-loaded chamber 506, this should not be construed as any limitation of the present disclosure. In some other embodiments, the device 500 may be provided with multiple microfluidic channels 504 and multiple top-loaded chambers 506 each connected to one or more of the microfluidic channels 504 (in this case, it is possible to perform the electrical stimulation and sensing of multiple different or same organoids in parallel). In some embodiments, the top-loaded chamber 506 may comprise its own one or more microfluidic channels for feeding a cell culture medium or fluid different than that passing through the microfluidic channel(s) 504.

It should be noted that, in practice, organoids and spheroids may be placed on the array 106 of needles present either in the replaceable inserts 306 of the cell culture plate 300 or in the top-loaded chamber 506 of the apparatus 500 as follows. Readymade or off-the-shelf organoids and spheroids may be picked using pipettes with appropriately sized tips to account for the organoid/spheroid diameter, and then dispensed gently on top of the needles 108. Alternatively, a cell suspension with or without an extracellular matrix or hydrogel, may be dispensed in the insert(s) 306 or the top-loaded chamber 506 containing the microneedles, allowing in-situ formation of organoids, spheroids or other 3D cell structures.

Although the exemplary embodiments of the present disclosure are described herein, it should be noted that any various changes and modifications could be made in the embodiments of the present disclosure, without departing from the scope of legal protection which is defined by the appended claims. In the appended claims, the word "comprising" does not exclude other elements, steps or operations, and the indefinite article "a" or "an" does not exclude a plurality. The mere fact that certain measures are recited in mutually different dependent claims does not indicate that a combination of these measures cannot be used to advantage.

## Claims

1. A membrane structure for stimulating and sensing a biological material, comprising:
a membrane having through pores formed therein, the membrane having a top surface and a bottom surface; and
at least one array of needles provided on at least one of the top surface and the bottom surface of the membrane such that the through pores of the membrane remain open;
wherein each needle of the at least one array of needles is shaped to penetrate the biological material when the biological material is provided on the at least one array of needles; and
wherein each needle of the at least one array of needles is made of a non-conducting material and at least partly coated with a conducting electrode.

2. The membrane structure of claim 1, wherein at least one needle of the at least one array of needles is fully coated with the conducting electrode.

3. The membrane structure of claim 1, wherein each needle of the at least one array of needles comprises a plurality of portions spaced from each other along or across the needle, each portion of the set of portions being coated with the conducting electrode.

4. The membrane structure of claim 3, wherein the plurality of portions has an equal inter-portion spacing.

5. The membrane structure of claim 3, wherein the plurality of portions has a varying inter-portion spacing.

6. The membrane structure of any one of claims 3 to 5, wherein the conducting electrode is configured as a ring surrounding each portion of the plurality of portions.

7. The membrane structure of any one of claim 1 to 6, wherein the at least one array of needles comprises a single array of needles each having a tapered shape, a mushroom-like shape, or a uniform cross-section shape.

8. The membrane structure of any one of claims 1 to 6, wherein the at least one array of needles comprises a first array of needles and a second array of needles, the first array of needles and the second array of needles differing from each other in at least one of: a needle size, a needle shape, and an inter-needle spacing.

9. The membrane structure of claim 8, wherein each of the first array of needles and the second array of needles has one of the following needle shapes: a tapered shape, a mushroom-like shape, and a uniform cross-section shape.

10. The membrane structure of claim 8 or 9, wherein the first array of needles and the second array of needles are provided on one of the top surface and the bottom surface of the membrane.

11. The membrane structure of claim 10, wherein the first array of needles and the second array of needles are non-overlapping.

12. The membrane structure of claim 10, wherein the first array of needles and the second array of needles are arranged alternately.

13. The membrane structure of claim 12, wherein each of the first array of needles and the second array of needles is a 2D array of needles, and wherein the first array of needles and the second array of needles are provided in a staggered arrangement.

14. The membrane structure of claim 8 or 9, wherein the first array of needles is provided on the top surface of the membrane, while the second array of needles is provided on the bottom surface of the membrane.

15. A cell culture plate comprising:
a housing encompassing a plurality of wells, each well of the plurality of wells being provided with a cell culture medium and a replaceable insert configured to be immersed into the cell culture medium, each of the replaceable inserts comprising the membrane structure according to any one of claims 1 to 14.

16. A microfluidic device comprising:
a housing having a microfluidic channel and a top-loaded chamber, the microfluidic channel being configured to pass a fluid or cell culture medium therethrough; and
the membrane structure according to any one of claims 1 to 14, the membrane structure being arranged in the housing such that the top-loaded chamber is in fluid communication with the microfluidic channel via the through pores of the membrane structure.

17. The device of claim 16, wherein the top-loaded chamber has an additional microfluidic channel formed therein, the additional microfluidic channel being configured to pass an additional fluid or cell culture medium therethrough.
